(19) Europäisches Patentamt

European Patent Office

Office européen des brevets

(11) Veröffentlichungsnummer: **0 358 970 B1**

(12) # EUROPÄISCHE PATENTSCHRIFT·

(45) Veröffentlichungstag der Patentschrift:
**18.12.91 Patentblatt 91/51**

(51) Int. Cl.⁵ : **A61K 31/575, A61K 31/58,**
**A61K 31/705, A61K 47/10,**
**A61K 47/28, A61K 9/10**

(21) Anmeldenummer: **89115148.2**

(22) Anmeldetag: **17.08.89**

(54) **Transdermal anwendbare pharmazeutische Zubereitungen mit Sterolen und/oder Spiroketalinen.**

(30) Priorität: **01.09.88 DE 3829641**

(43) Veröffentlichungstag der Anmeldung:
**21.03.90 Patentblatt 90/12**

(45) Bekanntmachung des Hinweises auf die
Patenterteilung:
**18.12.91 Patentblatt 91/51**

(84) Benannte Vertragsstaaten:
**AT BE CH ES FR GB IT LI LU NL SE**

(56) Entgegenhaltungen:
EP-A- 0 162 330
DE-A- 2 458 890
SEIFEN-OLE-FETTE-WACHSE, Band 109, Nr.
15, September 1983, Seiten 444-445, Verlag für
Chemische Industrie, H. Ziolkowsky KG, Augsburg, DE; C.-E. LOEVBERG: "Holzbasierendes
beta-Sitosterin-ein Rohstoff für die Kosmetikindustrie"

(56) Entgegenhaltungen:
**SEIFEN-OLE-FETTE-WASCHE, Band 112, Nr.
8, Mai 1986, Seiten 261-262, Verlag für Chemische Industrie, H. Ziolkowsky KG, Augsburg,
DE; A. HAMUNEN et al.: "Sterine aus unverseifbaren Bestandteilen des Tallöls - ein auf
Holz basierendes Rhohmaterial für die Kos-
metik-, Pharma- und Lebensmittelindustrie"**

(73) Patentinhaber: **Roecar Holdings (Netherlands
Antilles) N.V.
Kerkstraat 10a
Willemstad, Curacao Netherlands Antilles
(NL)**

(72) Erfinder: **Walker, Hans Dr.
Hessenring 29
W-3340 Eschwege (DE)**
Erfinder: **Pegel, Karl Heinrich Dr.
King George V Avenue
Durban, Natal (ZA)**

(74) Vertreter: **Siewers, Gescha, Dr.
Rechtsanwälte Dres. Harmsen, Utescher pp.
Adenauerallee 28
W-2000 Hamburg 1 (DE)**

**Beschreibung**

Die Erfindung betrifft neue transdermal anwendbare pharmazeutische Zubereitungen mit einem Gehalt an Sterolinen und/oder Spiroketalinen.

Als Steroline werden die Glykoside von Phytosterolen einschließlich Cholesterol bezeichnet. Phytosterole und ihre Glykoside kommen in der Natur in kleinen Mengen wahrscheinlich in allen Pflanzen und häufig auch in Mikroorganismen vor. Synthetisch können Steroline in der bekannten Königs-Knorr-Synthese unter Verwendung der entsprechenden Aglycone und eines am C-1 bromierten Zuckeracetates unter Verwendung von Silber- oder Kadmiumkatalysatoren in relativ großer Ausbeute hergestellt werden. Die Steroline kommen in der Natur meist als Monoglykoside vor, obgleich auch einige Oligoglykoside bekannt geworden sind. Darüber hinaus liegen die Steroline im natürlichen pflanzlichen Material zum Teil auch in Form der Ester, und zwar mit einbasischen Monocarbonsäuren, vor.

Als Sprioketaline bezeichnet man die von Steroidsaponinalkoholen abgeleiteten Mono- bis Triglykoside, die jeweils im Steroidgerüst des Aglycons eine an C-16 und C-17 angeschlossene Spiroketalgruppierung aufweisen. Die Aglycone können entweder zu den 5-En-steroidsapogeninen oder zu den 5-α-Steroidsapogeninen gerechnet werden. Beina türlichem Saponinmaterial liegen die Aglycone meist als Glykoside vor und enthalten 3 oder eventuell mehr Monosaccarideinheiten gekuppelt und können hydrolytisch mittels Enzymen zu Spiroketalinen abgebaut werden. Die Synthese der Spiroketaline nach Königs-Knorr ist ebenfalls in guten Ausbeuten möglich. Es ist bereits seit langem bekannt, daß sowohl Steroline als auch Spiroketaline eine Vielzahl von pharmakologischen Aktivitäten aufweisen, da sie, wie aus der GB-PS 2039217 bekannt ist, sowohl in den Prostaglandinsynthetasen- als auch, wie beispielsweise aus der DE-OS 3416112 bekannt, in den Lipoxygenasenspiegel eingreifen. Steroline und Spiroketaline können daher nicht nur zur Behandlung entzündlicher Erscheinungen, sondern auch überall dort eingesetzt werden, wo eine Normalisierung des Arachidonsäurehaushaltes notwendig ist, also beispielsweise bei Asthma, Akne, Psoriasis, Weichteil-Rheumatismus, Magen- und Darmgeschwüren, Thrombophlebitiden.

Bei der bisherigen Anwendung von insbesondere Sterolinen hat sich herausgestellt, daß hervorragende Ergebnisse bei in vitro-Versuchen sich nicht in vivo reproduzieren lassen. So haben J. Seki et al in J. Pharm Sci., 1985, 74, 1259 pp eine intestinale Absorption von Sitosterolglykosid von nur 1-2% festgestellt. Dies liegt häufig daran, daß sich aufgrund der extrem schlechten Wasserlöslichkeit der Steroline und der schlechten Wasserlöslichkeit der Spiroketaline keine ausreichenden Resorptionen und damit Blutspiegel erzielen lassen. Es ist daher auch schon versucht worden, die Resorption beispielsweise durch Verringerung der Teilchengröße, durch Anwendung von besser wasserlöslichen Derivaten wie Hemiestern oder durch die Verwendung von Lösungsvermittlern, wie beispielsweise in der US-PS 3966918 angegeben, zu verbessern. Überprüfungen mit $^{14}$C-markierter Substanz zeigen aber deutlich daß durch Mikronisierung oder andere Verringerung der Teilchengröße die Resorption nur geringfügig angehoben werden kann und daß dies ebenfalls zutrifft, wenn man versucht, mit Hilfe von Lösungsvermittlern den Anteil der Verbindungen in der wässrigen Phase zu erhöhen.

Es besteht daher immer noch ein Bedürfnis nach pharmazeutischen Zubereitungen mit einem Gehalt an Sterolinen und/oder Spiroketalinen, die über eine bessere Resorptivität verfügen.

Erfindungsgemäß werden nunmehr transdermal anzuwendende pharmazeutische Zubereitungen vorgeschlagen, die einen Gehalt an Sterolinen und/oder Spiroketalinen zusammen mit Ethylenoxidaddukten mit 20 bis 30 Ethylenoxideinheiten an Sterole oder Alkoholen enthalten.

Völlig überraschend wurde nunmehr festgestellt, daß es möglich ist, die nicht nur in Wasser, sondern in der Regel auch in lipophilen Lösungsmitteln schwer löslichen Steroline und Spiroketaline mit Hilfe von Lösungsvermittlern in einer Ölphase zu lösen und hieraus, auch wenn diese in Form von Emulsionen vorliegt, transdermal zur Resorption zu bringen, und zwar zu einer weitaus höheren Resorption als bisher jemals auf oraler oder intravasaler Route möglich.

Erfindungsgemäß werden die Wirkstoffe zusammen mit ethoxilierten Sterolen oder C12-C30 Alkoholen, die jeweils mit Ethylenoxid umgesetzt wurden, vermischt und in mehrwertigen Alkoholen wie 1,2-Propandiol oder Glycerin unter leichtem Erwärmen gelöst. Als Lösungsvermittler und Schlepper bei der späteren transdermalen Resorption können alle ethoxilierten Sterole oder C12-C30 Alkohole verwendet werden, und zwar im Gemisch oder als Monoverbindung. Diese durch Umsetzen der freien Alkohole mit Ethylenoxid unschwer herstellbaren Verbindungen sind teilweise bereits als Lösungsvermittler bekannt und im Handel wie beispielsweise die unter der Bezeichnung "Generol-E" von der Firma Henkel KGaA vertriebenen Produkte. Es handelt sich meist um Sitosterol oder Sterolgemische mit einem Ethoxylierungsgrad zwischen etwa 20 bis 30. Bevorzugt eingesetzt wird ein ethoxyliertes Sitosterol mit einer Kettenlänge von 25 EO-Einheiten. Als Alkohole mit 12-30 C-Atomen können insbesondere Fettalkohole, aber auch natürlich vorkommende verzweigt, alicyclische oder aromatische Alkohole verwendet werden wie z.B. Derivate des Squalens oder Phytol.

Die Lösungen von Sterolinen oder Spiroketalinen in Lösungsvermittler und mehrwertigem Alkohol lösen sich wiederum klar in Ölen oder geschmolzenen Fetten. Hier kommen vor allen Dingen die üblichen Salbengrundlagen in Frage wie beispielsweise mittelkettige Triglyceride, Glycerolmonostearat, Wollwachs, Schweineschmalz. Völlig überraschend hat sich auch herausgestellt, daß man nach dem Einarbeiten der Wirksubstanzen in die Fettgrundlagen diese zu Emulsionssalben weiterverarbeiten kann, da sich die Mischung ohne Schwierigkeiten mit der gleichen Menge Wasser emulgieren läßt. Unabhängig davon, ob die Wirkstoffe in einer wasserfreien oder wasserhaltigen Fettgrundlage vorliegen, zeigen Untersuchungen mit $^{14}$C markiertem Wirkstoff, daß Penetration in die Haut und Permeation in etwa gleicher Größenordnung folgen. Die Wirkstoffe dringen dabei nicht nur in die Haut ein, sondern permeieren sogar durch die Haut hindurch, so daß sich bei großflächigerem Auftragen Plasmaspiegel in einer Höhe erreichen lassen, die auf intestinalem Weg nicht zu erzielen ist. Überraschenderweise besteht auch hinsichtlich der Transdermalresorption trotz unterschiedlicher chemischer Struktur zwischen Sterolinen und Spiroketalinen kaum ein Unterschied. Wie die $^{14}$C Untersuchungen zeigen, sind 24 Stunden nach dem Auftragen etwa 10% der Wirkstoffe durch die Haut permeiert und etwa 6% in die Haut penetriert.

Die durchschnittliche Zusammensetzung für die transdermale Applikation beträgt 0,1% Wirkstoff, 1% Ethylenoxidaddukte und 10% mehrwertigem Alkohol. Nach dem Lösen wird die klare Lösung entweder mit der entsprechenden Menge wasserfreier Salbengrundlage vermischt oder in an und für sich bekannter Weise mit etwa der halben Menge Salbengrundlage mit Zusatz der üblichen Menge nichtiogener Emulgatoren sowie Wasser versetzt und in üblicher Weise unter Rühren mit der Restmenge emulgiert.

Bei der klinischen Prüfung haben sich mit den erfindungsgemäßen pharmazeutischen Zubereitungen besonders schnelle und überzeugende Wirkungen bei solchen Erkrankungen aufzeigen lassen, bei denen ein erhöhter Leukotrienspiegel Ursache oder zumindest Mediator des pathologischen Geschehens ist wie beispielsweise bei Allergien, Ekzemen, chronischem Jukreiz, bestimmten Psoriasisformen, Akne, aber auch bei rheumatoiden Erkrankungen und Thrombophlebitiden.

Die Erfindung wird im folgenden anhand der Beispiele näher erläutert :

## Beispiel 1

0,1 g Cholesteringlykosid, 80,0 g ethoxilierter Palmityl-Stearyl-Alkohol werden in 100,0 g 1,2-Propandiol gelöst. Diese auf 70°C erwärmte Lösung wird dann zu einer Schmelze aus 120,0 g Adeps solidus, 100,0 g mittelkettige Triglyceride und 120 g Glycerinmonostearat bei einer Temperatur von 70°C zugegeben um mit ebenfalls angegebene Temperatur erwärmten Wasser bis zu einer Gesamtmenge von 1000,0 g emulgiert. Die Emulsion wird danach kaltgerührt.

## Beispiel 2

1,0 g Diosgeninglykosid wird in einer Mischung aus 10,0 g ethoxiliertem Sojasterin (25 EO-Einheiten) und 100,0 g 1,2-Propandiol gelöst. Diese auf 70°C erwärmte Lösung wird dann zu einer ebenfalls auf 70°C erwärmten Schmelze aus 100,0 g Cetylpalmitat, 250,0 g Stearylheptanoat, 100,0 g Sorbitanmonostearat und 60,0 g Polyäthylensorbitan-monostearat zugegeben und mit ebenfalls auf 70°C erwärmten Wasser auf eine Gesamtmenge von 1000,0 g emulgiert. In an und für sich bekannter Weise wird die Emulsion dann kaltgerührt.

## Beispiel 3

1,0 g Sitosterolglykosid wird in 10,0 g ethoxiliertem Sojasterin (25 EO-Einheiten) und 100,0 g 1,2-Propandiol gelöst und bei den angegebenen Temperaturen zu einer Schmelze aus 300,0 g hydriertem Kokosöl, 50,0 g mittelkettigen Triglyceriden, 120,0 g Glycerinmonostearat und 80,0 g ethoxiliertem Cetylstearylalkohol (25 EO-Einheiten) zugegeben. Emulgiert wird aus 70°C erwärmten Wasser zu einer Gesamtmenge von 1000,0 g. Die Emulsion wird dann wie üblich kaltgerührt.

## Beispiel 4

1,0 g Sitosterolglykosid wird in 10,0 g ethoxiliertem Sojasterin (25 EO-Eineheiten) und 100,0 g 1,2-Propandiol gelöst und bei den angegebenen Temperaturen zu einer Schmelze aus 300,0 g hydriertem Kokosöl, 50,0 g mittelkettigen Triglyceriden, 120,0 g Glycerinmonosstearat und 80,0 g ethoxiliertem Cetylstearylalkohol (25 EO-Einheiten) zugegeben. Emulgiert wird aus 70°C erwärmten Wasser zu einer Gesamtmenge von 1000,0 g. Die Emulsion wir dann wie üblich kaltgerührt.

## Beispiel 5

Penetration — bzw. Permeationsmessungen

Als Versuchstiere werden männliche, ca. 10 Wochen alte Kaninchen mit einem Körpergewicht von etwa 2 kg verwendet. Auf einer rasierten Fläche von 10 × 5 cm auf dem Rücken eines Kaninchens werden 1,0 g Creme mit einer Radioaktivität von 20 μ Ci des jeweils ($^{14}$C-4) markiertem Glykosides aufgetragen, mit Alufolie abgedeckt und mit wasserfestem Heftpflaster fixiert. Nach 24 Stunden werden die Kaninchen eingeschläfert und der Applikationsbereich wird mehrmals mit ethanolfeuchten Tupfern gewaschen.

Die Radioaktivität des Okklusivverbandsmaterials und der Tupfer wird nach Extraktion mit Ethanol gemessen. Die Haut des Anwendungsbereichs wird mit 2 m Natronlauge/Methonaol/Triton X 405 (6 : 3 : 1 v/v) bei 55°C während 24 Stunden behandelt und danach mit 40°C warmen Ethanol gewaschen. Die Radioaktivität gemessen mit einem Flüssigkeits-Scintillationszähler (Philips PW 4700) ist ein Maß für die prozentuale Penetration der applizerten Substanzmenge. Die Permeation während 24 Stunden wird wie folgt berechnet : 100% − (Aktivität der Waschflüssigkeit + Aktivität der Penetrationsmenge) = % Permeation. Unter den angegebenen Testbedingungen ergab sich bei Cholesterolglykosid eine Penetration von 4,9% der applizierten Dosis und eine Permeation von 7,8% der applizierten Dosis. Für Diosgeninglykosid ergaben sich 5,2 bzw. 10,3% und für Sitosterolglykosid lauten die Zahlen 6,2 bzw. 10,6%.

## Patentansprüche

1. Transdermal anwendbare pharmazeutische Zubereitungen mit Sterolinen und/oder Spiroketalinen, gekennzeichnet durch die Verwendung von ethoxilierter Sterole und/oder C12-C30 Alkohole als Lösungsmittel in lipophiler Phase.

2. Pharmzeutische Zubereitungen nach Anspruch 1, gekennzeichnet durch einen Zusatz von Sterolen und/oder C12-C30 Alkoholen mit 20-30 EO-Einheiten.

3. Pharmazeutische Zubereitungen nach Anspruch 1 oder 2, gekennzeichnet, durch einen Zusatz von Sitosterol mit 25 EO-Einheiten.

4. Pharmazeutische Zubereitungen nach Anspruch 1-3, dadurch gekennzeichnet, daß die lipophile Phase als W/O-Emulsion vorliegt.

## Claims

1. Transdermally applicable pharmaceutical compositions containing sterolins and/or spiroketalins, characterized by the use of ethoxylated sterols and/or C12-C30 alcohols as solvents in the lipophilic phase.

2. Pharmaceutical compositions according to claim 1, characterized by the addition of sterols and/or C12-C30 alcohols with 20-30 EO units.

3. Pharmaceutical compositions according to claim 1 or 2, characterized by the addition of sitosterol with 25 EO units.

4. Pharmaceutical preparations according to claim 1-3, characterized in that the lipophilic phase is present in the form of a water-in-oil emulsion.

## Revendications

1. Compositions pharmaceutiques contenant des stérols et/ou spirokétalines pour l'usage transcutané, caractérisées par l'utilisation de stérols éthoxiliés et/ou d'alcools C12 à C30 comme solvants dans la phase lipophile.

2. Compositions pharmaceutiques selon la revendication 1, caractérisées par l'ajoutage de stérols et/ou d'alcools C12 à C30 avec 20 à 30 unités EO.

3. Compositions pharmaceutiques selon la revendication 1 ou 2, caractérisées par l'ajoutage de sitostérol avec 25 unités EO.

4. Compositions pharmaceutiques selon les revendications 1 à 3, caractérisées par le fait que la phase lipophile se présente comme une émulsion type eau-en-huile.